# EUROPÄISCHE PATENTSCHRIFT

(11) **EP 2 015 811 B1**
(45) Veröffentlichungstag und Bekanntmachung des Hinweises auf die Patenterteilung: **06.10.2010**
(21) Anmeldenummer: 07724260.0
(22) Anmeldetag: 16.04.2007
(51) Int. Cl.: A61M 15/00, B05B 11/00

(54) **ZERSTÄUBER UND VERFAHREN ZUR ZERSTÄUBUNG VON FLUID**
ATOMIZER AND METHOD OF ATOMIZING FLUID
PULVÉRISATEUR ET PROCÉDÉ DE PULVERISATION D'UN FLUIDE

(30) Priorität: 10.05.2006 DE 102006022002
(43) Veröffentlichungstag der Anmeldung: 21.01.2009
(73) Patentinhaber: Boehringer Ingelheim International GmbH, 55216 Ingelheim (DE)
(72) Erfinder: BOECK, Georg, 88471 Laupheim (DE); SPALLEK, Michael, 55218 Ingelheim (DE)
(74) Vertreter: Hammann, Heinz
(86) Internationale Anmeldenummer: PCT/EP2007/003322
(87) Internationale Veröffentlichungsnummer: WO 2007/128381

(56) Entgegenhaltungen:
- WO-A-98/12511
- US-A1- 2005 183 718
- US-B1- 6 401 987
- US-B1- 6 402 055

## Beschreibung

Die vorliegende Erfindung betrifft einen Zerstäuber gemäß dem Oberbegriff des Anspruchs 1 und ein Verfahren gemäß dem Oberbegriff des Anspruchs 12, Zerstäuber und Verfahren beide von Dokument US20050183718 offenbart.

Bekannt ist ein unter dem Handelsnamen "Respimat" angebotener Zerstäuber in Form eines Inhalators, wie im Grundprinzip in der WO 91/14468 A1 und in konkreter Ausgestaltung in der WO 97/12687 A1 (Fig. 6a, 6b) sowie in Fig. 1 und 2 der anliegenden Zeichnung dargestellt. Der Zerstäuber weist als Reservoir für ein zu zerstäubendes Fluid einen einsetzbaren Behälter, einen Druckerzeuger mit einer Antriebsfeder sowie eine Düse, über die das Fluid ausgegeben und zerstäubt wird, auf.

Nach dem erstmaligen Einsetzen des Behälters oder längerem Nichtgebrauch kann Luft im Druckerzeuger bzw. Düsensystem dazu führen, daß bei der nächsten Ausgabe und Zerstäubung von Fluid die Dosiergenauigkeit nachteilig beeinflußt wird. Dies ist bei der darauf folgenden Ausgabe und Zerstäubung nicht mehr der Fall, da eventuell vorhandene Luft durch Fluid verdrängt wurde. Vor dem erstmaligem Gebrauch des Zerstäubers und nach längerem Nichtgebrauch des Zerstäubers wird daher empfohlen, vor der eigentlichen Benutzung des Zerstäubers zunächst mindestens einmal eine Fluidausgabe ohne Inhalation durchzuführen. Diese Vorabbetätigung wird auch als "Primen" bezeichnet. Das Primen führt zu einem erhöhten Fluidverbrauch und erfordert besondere Aufmerksamkeit des Benutzers.

Der vorliegenden Erfindung liegt die Aufgabe zugrunde, einen Zerstäuber und ein Verfahren sowie eine Vewendung eines derartigen Zerstäubers anzugeben, so daß auf einfache Weise und bei einfacher Handhabung eine besonders genaue Dosierung ermöglicht wird, insbesondere ein Primen entfallen kann.

Die obige Aufgabe wird durch einen Zerstäuber gemäß Anspruch 1 oder ein Verfahren gemäß Anspruch 12 gelöst. Vorteilhafte Weiterbildungen sind Gegenstand der Unteransprüche.

Eine grundlegende Idee der vorliegenden Erfindung liegt darin, daß jeweils eine die Düse und ggf. sonstige Fördereinrichtungen des jeweiligen Zerstäubers spülende - im Vergleich zu jeder Dosis jedoch sehr geringe - Vormenge an Fluid vorzugsweise zwangsweise vor jeder bestimmungsgemäßen Ausgabe einer Dosis oder bei jedem Spannen des Zerstäubers ausgegeben wird. Durch den Begriff "bestimmungsgemäß" wird klargestellt, daß gegebenenfalls bei dem erstmaligen Primen, dem sogenannten Trockenprimen, des Zerstäubers keine Ausgabe einer Dosis beabsichtigt ist bzw. erfolgt und dabei ebenfalls keine Vormenge ausgegeben wird, insbesondere wenn anfänglich nur Luft aus dem Fördersystem des Zerstäubers verdrängt wird. Die Ausgabe bzw. Erzeugung des Vorsprays führt zu mehreren Vorteilen.

Das Spülen mit der Vormenge kann die Dosiergenauigkeit des Zerstäubers weiter verbessern.

Die in dem Druckerzeuger oder dergleichen enthaltene Luft und sonstige Gase werden durch die Vormenge verdrängt oder zumindest wesentlich reduziert. Dies verbessert die Dosiergenauigkeit bei der nachfolgenden Ausgabe.

Durch die Vormenge kann beispielsweise ein definiertes Schließen oder sonstiges Einnehmen einer bestimmten Position eines Ventils insbesondere des Druckerzeugers erreicht werden, so daß definiertere Zustände bei der eigentlichen Benutzung des Zerstäubers und Dosierung des insbesondere für die Inhalation vorgesehenen Fluids erreichbar sind.

Weiter kann an der Düse ein definierter Fluidabriß durch die Vormenge erreicht werden, der im wesentlichen demjenigen bei der unmittelbar nachfolgenden Ausgabe von Fluid entspricht, wodurch wiederum die Dosiergenauigkeit weiter verbessert wird.

Das Spülen der Düse durch die Vormenge kann Verlegungen, Ablagerungen, Verschmutzungen oder mikrobielle Verunreinigungen austragen bzw. auflösen.

Auch können Konzentrationszunahmen - beispielsweise aufgrund von Verdunstung während der Nichtbenutzung - vermindert oder vermieden werden.

Bei der vorliegenden Erfindung bezieht sich der Begriff "Spülen der Düse" vorzugsweise nicht nur auf die Düse oder das Düsensystem des Zerstäubers, sondern bezieht sich alternativ oder zusätzlich auch auf den Druckerzeuger oder sonstige Teile des Zerstäubers zur Förderung, Ausgabe und Zerstäubung von Fluid, wie ein eventuell vorhandener Filter, eine Druckkammer, ein Ventil, eine Ansaugleitung, eine Druckleitung oder dergleichen.

Falls oligodynamische Silberverbindungen oder andere Ionen freisetzende Verbindungen im Zerstäuber eingesetzt werden, können die Silberionen bzw. sonstigen Ionen durch die Vormenge ausgetragen werden, so daß weniger Ionen bei der eigentlichen Benutzung ausgegeben und inhaliert werden.

Das vorschlagsgemäße Spülen durch die Vormenge erfolgt insbesondere selbsttätig, also ohne separate Betätigung oder Auslösung durch den Benutzer, insbesondere bei jedem Spannen des Zerstäubers bzw. einer im Zerstäuber enthaltenen Feder oder dergleichen. Dementsprechend ergibt sich eine sehr einfache Bedienung. Besonders bevorzugt kann das genannte Primen ganz entfallen oder zumindest verringert werden.

Es wurde festgestellt, daß eine überraschend geringe Vormenge genügt, insbesondere um die vorgenannten Vorteile zu erreichen. Auch wenn die Düse bei jeder Betätigung bzw. Benutzung des Zerstäubers durch die Vormenge gespült wird, ist der Fluidverbrauch insgesamt voraussichtlich geringer, da das Primen - auch wenn es nicht vor jeder Benutzung des Zerstäubers erfolgt - jeweils zum Austrag einer vollen Dosis an Fluid führt.

Die Vormenge an Fluid wird insbesondere je nach Druck über die Düse als Sprühnebel und/oder als Tropfen ausgegeben. Nachfolgend wird das Spülen der Düse durch die Vormenge und deren Ausgabe durch die Düse - unabhängig davon, ob als Sprühnebel oder Tropfen - auch als "Vorspray" bezeichnet.

Weitere Vorteile, Merkmale, Eigenschaften und Aspekte der vorliegenden Erfindung ergeben sich aus den Ansprüchen und der folgenden Beschreibung einer bevorzugten Ausführungsform anhand der Zeichnung. Es zeigt:
- Fig. 1: einen schematischen Schnitt eines bekannten Zerstäubers im un- gespannten Zustand;
- Fig. 2: einen schematischen, um 90° gegenüber Fig. 1 gedrehten Schnitt des bekannten Zerstäubers im gespannten Zustand;
- Fig. 3: einen schematischen Schnitt des vorschlagsgemäßen Zerstäubers.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

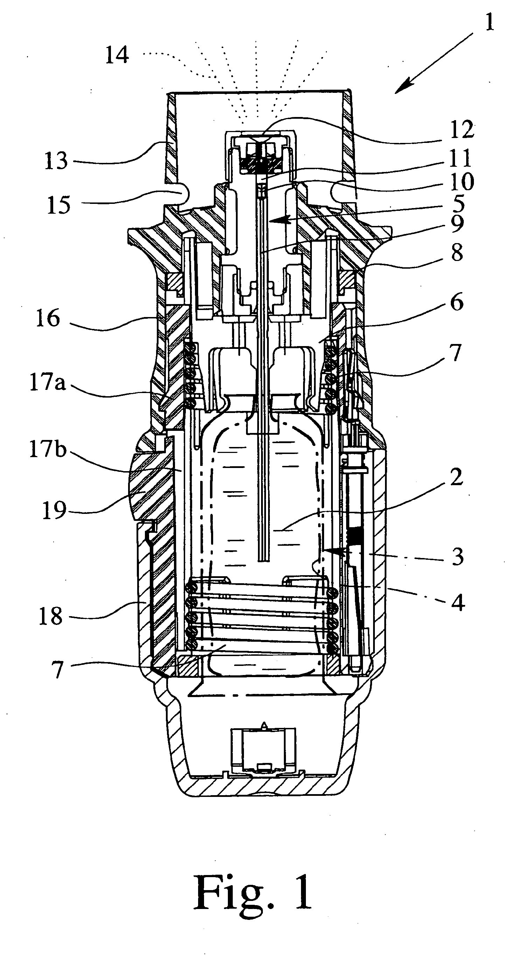

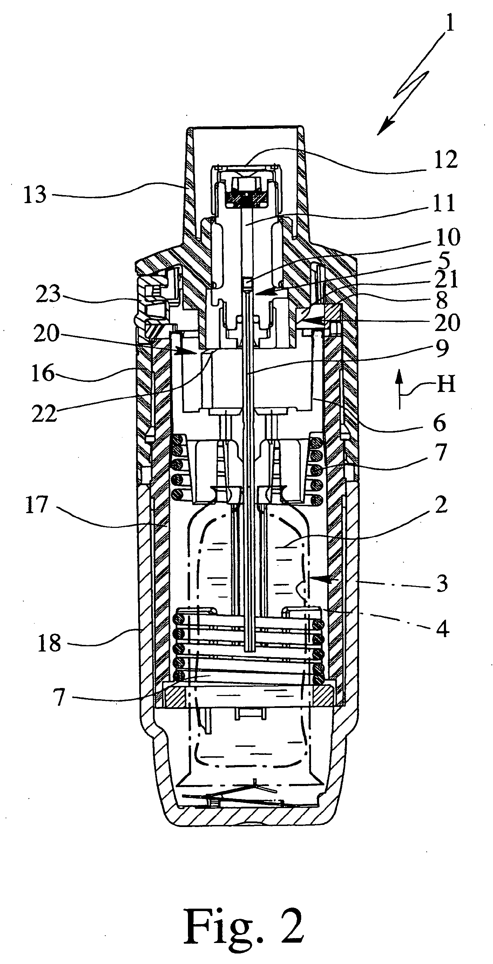

Fig. 1 und 2 zeigen einen bekannten Zerstäuber 1 zur Zerstäubung eines Fluids 2, insbesondere eines hochwirksamen Arzneimittels oder dgl., in einer schematischen Darstellung im ungespannten Zustand (Fig. 1) und gespannten Zustand (Fig. 2). Der Zerstäuber 1 ist insbesondere als tragbarer Inhalator ausgebildet und arbeitet vorzugsweise ohne Treibgas.

Bei Zerstäubung des Fluids 2, vorzugsweise einer Flüssigkeit, insbesondere eines Arzneimittels, wird ein Aerosol gebildet, das von einem nicht dargestellten Benutzer eingeatmet bzw. inhaliert werden kann. Üblicherweise erfolgt das Inhalieren wenigstens einmal täglich, insbesondere mehrmals täglich, vorzugsweise in vorbestimmten Zeitabständen, in Abhängigkeit von der Erkrankung des Patienten.

Der bekannte Zerstäuber 1 weist einen vorzugsweise einsetzbaren und vorzugsweise wechselbaren Behälter 3 mit dem Fluid 2 auf. Der Behälter 3 bildet also ein Reservoir für das zu zerstäubende Fluid 2, das insbesondere in einem von einem kollabierbaren Beutel gebildeten Fluidraum 4 im Behälter 3 aufgenommen ist.

Vorzugsweise enthält der Behälter 3 eine ausreichende Menge an Fluid 2 bzw. Wirkstoff, um beispielsweise bis zu 200 Dosiereinheiten bzw-. Dosen zur Verfügung stellen zu können, also beispielsweise bis zu 200 Zerstäubungen oder Anwendungen zu ermöglichen. Ein typischer Behälter 3, wie in der WO 96/06011 A2 offenbart, nimmt ein Volumen von ca. 2 bis 10 ml auf.

Der Zerstäuber 1 weist ferner einen Druckerzeuger 5 zur Förderung und Zerstäubung des Fluids 2, insbesondere jeweils in einer vorbestimmten oder einstellbaren Dosiermenge, auf. Das Fluid 2 ist also in einzelnen Dosen, insbesondere von jeweils etwa 5 bis 30 µl, ausgebbar und zerstäubbar.

Der Druckerzeuger 5 weist eine Halterung 6 für den Behälter 3, eine zugeordnete, nur teilweise dargestellte Antriebsfeder 7, ein zur Entsperrung vorzugsweise manuell betätigbares Sperrelement 8, ein bewegbares Förderelement, insbesondere ein Förderrohr 9, ein Rückschlagventil 10, eine Druckkammer 11 und eine Düse 12 im Bereich eines Mundstücks 13 auf. Der Behälter 3 wird über die Halterung 6, insbesondere rastend, so in dem Zerstäuber 1 fixiert, daß das Förderrohr 9 in den Behälter 3 eintaucht. Die Halterung 6 kann dabei derart ausgebildet sein, daß der Behälter 3 gelöst und ausgetauscht werden kann.

Zum axialen Spannen der Antriebsfeder 7 wird die Halterung 6 mit dem Behälter 3 und dem Förderrohr 9 bei den Darstellungen nach unten bewegt und Fluid 2 aus dem Behälter 3 über das Rückschlagventil 10 in die Druckkammer 11 des Druckerzeugers 5 gesaugt.

Beim anschließenden Entspannen nach Betätigung des Sperrelements 8 zur Freigabe der Halterung 6 wird das Fluid 2 in der Druckkammer 11 unter Druck gesetzt, indem das Förderrohr 9 mit seinem nun geschlossenen Rückschlagventil 10 durch Entspannen der Antriebsfeder 7 wieder nach oben bewegt wird (Hauptbewegung), also als Druckstempel arbeitet. Dieser Druck treibt das Fluid 2 durch die Düse 12 aus, wobei es in ein Aerosol 14 zerstäubt wird, wie in Fig. 1 angedeutet.

Ein nicht dargestellter Benutzer bzw. Patient kann das Aerosol 14 inhalieren, wobei Zuluft über mindestens eine Zuluftöffnung 15 in das Mundstück 13 saugbar ist.

Der Zerstäuber 1 weist ein Gehäuseoberteil 16 und ein demgegenüber drehbares Innenteil 17 (Fig. 2) mit einem oberen Teil 17a und einem unteren Teil 17b (Fig. 1) auf, wobei an dem Innenteil 17 ein insbesondere manuell betätigbares Gehäuseteil 18 vorzugsweise mittels eines Halteelementes 19 lösbar befestigt, insbesondere aufgesteckt, ist. Zum Einsetzen und/oder Auswechseln des Behälters 3 ist das Gehäuseteil 18 vom Zerstäuber 1 lösbar.

Das Gehäuseteil 18 kann relativ zum Gehäuseoberteil 16 gedreht werden, wobei es den Innenteil 17 mitnimmt. Der obere Teil 17a dreht durch entsprechenden Eingriff die Halterung 6, so daß diese über ein in Fig. 2 nur angedeutetes Getriebe 20 gegen die Kraft der Antriebsfeder 7 axial verschoben und die Antriebsfeder 7 gespannt wird.

Beim Spannen wird der Behälter 3 axial nach unten bewegt. Schließlich nimmt der Behälter 3 eine in Fig. 2 angedeutete Endlage. In dieser Endlage ist die Antriebsfeder 7 gespannt. Das Sperrelement 8 kann dann die Halterung 6 und damit das Förderrohr 9 und den Behälter 3 gegen eine Bewegung in der Darstellung nach oben, also ein Entspannen der Antriebsfeder 7 sperren. In der Darstellung gemäß Fig. 2 befinden sich die Halterung 6 mit dem Förderrohr 9 und dem Behälter 3 und die Antriebsfeder 7 in dem gespannten Zustand bzw. in der Spannposition, das Sperrelement 8 befindet sich jedoch nicht in seiner die Halterung 6 blockierenden, quer bzw. radial verschobenen Sperrlage.

Nach dem Freigeben der Halterung 6 durch das Sperrelement 8 - also ausgehend von der in Fig. 2 gezeigten Spannposition und bei der in Fig. 2 gezeigten Lage des Sperrelements 8 - erfolgt der Zerstäubungsvorgang. Hierbei wird die Halterung 6 zusammen mit dem Förderrohr 9 und dem Behälter 3 wieder in die in Fig. 1 gezeigte Ausgangsposition durch die Kraft der Antriebsfeder 7 zurückbewegt. Diese Bewegung wird nachfolgend auch kurz als Hauptbewegung H bezeichnet. Bei der Hauptbewegung ist das Ventil 10 geschlossen und setzt das Förderrohr 9 in der Druckkammer 11 befindliches Fluid 2 unter Druck, so daß dieses über die Düse 12 ausgegeben und zerstäubt wird.

Der Behälter 3 führt also vorzugsweise eine Hubbewegung während des Spannvorgangs bzw. zur Fluidentnahme und während des Zerstäubungsvorgangs aus.

Das Getriebe 20 zum Spannen des Zerstäubers 1 bzw. der Antriebsfeder 7 und zum axialen Bewegen der Halterung 6 in Spannrichtung weist beim Darstellungsbeispiel vorzugsweise Abgleitflächen 21 und 22 am Gehäuseoberteil 18 und/oder an der Halterung 6 auf, die insbesondere schraubenlinig verlaufen und beim Drehen der Halterung 6 zum Gehäuseoberteil 16 zu der gewünschten Axialbewegung der Halterung 6 führen.

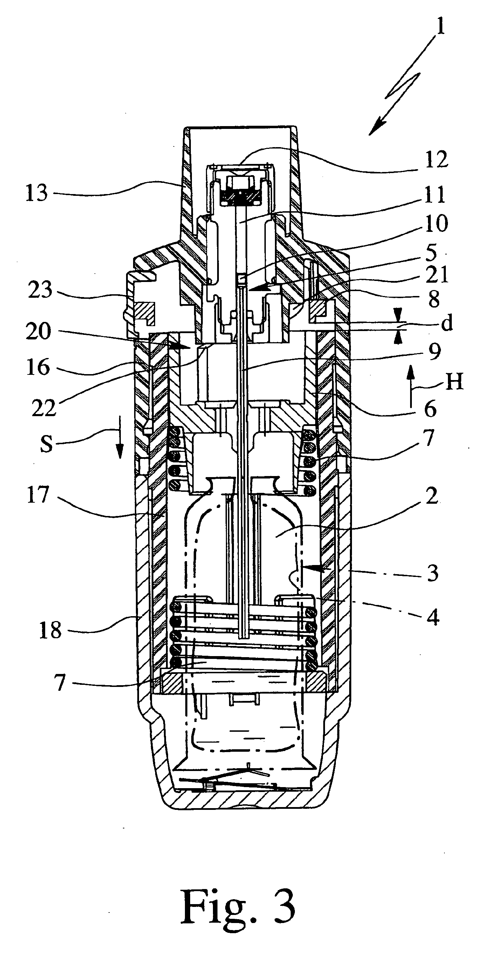

Wenn die Halterung 6 die in Fig. 2 dargestellte Spannposition erreicht, treten die Gleitflächen 21, 22 außer Eingriff und gibt das Getriebe 20 die Halterung 6 für die entgegengesetzte Bewegung in axialer Richtung, insbesondere die Hauptbewegung, frei. Gleichzeitig wird das Sperrelement 8 quer, insbesondere radial, zur Axialbewegung bzw. -richtung derart in seine Sperrlage (in Fig. 3 gezeigt) verlagert, daß die Halterung 6 und damit auch das Förderrohr 9 und der Behälter 3 in der Spannposition blockiert bzw. gesperrt werden. Insbesondere ist das Sperrelement 8 ringförmig ausgebildet und in seiner Sperrlage aus der normalerweise konzentrischen Anordnung zur Halterung 6 radial verschoben, so daß die Halterung 6 stirnseitig an einem Abschnitt des Sperrelements 8 anliegt und dadurch blockiert ist.

Zum Auslösen des Zerstäubungsvorgangs wird das insbesondere mit einer Taste 23 oder einem sonstigen Betätigungselement versehene Sperrelement 8 betätigt und dadurch wieder radial in seine konzentrische Lage, die in Fig. 2 dargestellt ist, zurückbewegt, wodurch die Blockierung der Halterung 6 aufgehoben wird und diese die Hauptbewegung in Richtung des Pfeils H ausführen kann. Dabei wird dann in der Druckkammer 11 befindliches Fluid 2 über die Düse 12 ausgegeben und zerstäubt.

Nachfolgend werden der Aufbau und die Funktionsweise eines vorschlagsgemäßen Zerstäubers 1 anhand der schematischen, nicht maßstabsgerechten Schnittdarstellung gemäß Fig. 3 erläutert, die im wesentlichen der Darstellung gemäß Fig. 2 entspricht. Die bisherigen Ausführungen, insbesondere zu Fig. 1 und 2, gelten entsprechend bzw. ergänzend.

Der vorschlagsgemäße Zerstäuber 1 ist derart ausgebildet, daß vor jeder Ausgabe und Zerstäubung einer Dosis an Fluid 2 eine bestimmte Vormenge an Fluid 2 ausgegeben wird. Diese Vormenge spült die Düse 12 bzw. sonstige Fördereinrichtungen, -komponenten oder dergleichen des Zerstäubers 1, insbesondere das Förderrohr 9, das Ventil 10, die Druckkammer 11, einen zur Düse 12 führenden Kanal, einen optionalen Filter vor der Düse 12, die Düse 12 oder dergleichen. Das Spülen mit der Vormenge erfolgt insbesondere zwangsweise bei jeder Betätigung des Zerstäubers 1, insbesondere bei jedem Spannen des Zerstäubers 1, und führt zu dem Vorspray und den bereits genannten Vorteilen.

Die Vormenge ist vorzugsweise sehr gering, bedarf aber einer gewissen Mindestmenge zur Erzielung der gewünschten Vorteile. Sie beträgt vorzugsweise mindestens 0,5 µl, insbesondere 0,5 bis 3 µl, besonders bevorzugt etwa 1 bis 2 µl. Dies ist im Vergleich zu der üblichen Dosis (insbesondere etwa 15 bis 20 µl), die bei jedem Zerstäubungsvorgang an Fluid 2 ausgegeben wird, sehr gering. Vorzugsweise beträgt die Vormenge mindestens 1 %, insbesondere mindestens 2 %, ganz bevorzugt etwa 3 bis 10 %, der Menge einer Dosis des Fluids 2, die während eines normalen Zerstäubungsvorgangs ausgegeben wird.

Besonders bevorzugt beträgt die Vormenge mindestens das 5-fache des Volumens der Düse 12 bzw. eines die Düse 12 bildenden Düsenblocks. Dies garantiert bereits ein effektives Spülen der Düse 12.

Nachfolgend wird erläutert, wie die Vormenge und das Spülen mit der Vormenge an Fluid 2 bevorzugt realisiert werden.

Beim Spannen des Zerstäubers 1 bzw. der Antriebsfeder 7 in Spannrichtung S werden die Halterung 6 und damit das Förderelement 9 in eine in Fig. 3 dargestellte Überspannposition bewegt. In dieser Überspannposition gibt das Getriebe 20 die Halterung 6 frei, so daß dann durch die Federkraft eine entgegengesetzte Bewegung - wie beim normalen Zerstäubungsvorgang - einsetzen kann. Die vorliegende Erfindung sieht vor, daß nun eine erste Bewegung - nachfolgend Vorbewegung genannt - in dieser Richtung H erfolgt, jedoch zunächst nur bis zum Erreichen der (normalen) Spannposition, d.h. beim Darstellungsbeispiel bis zur Anlage der Halterung 6 am sich in der Sperrlage befindenden Sperrelement 8. Fig. 3 zeigt das Sperrelement 8 in der Sperrlage mit ausgerückter Taste 23. Fig. 2 zeigt das Sperrelement 8 in der Nicht-Sperrlage, also in der die Halterung 6 nicht blockierenden Lage mit eingerückter Taste 23 bzw. in konzentrischer Lage.

Die Halterung 6 und damit auch das Förderelement bzw. Förderrohr 9 führen selbsttätig aus der in Fig. 3 dargestellten Überspannposition die Vorbewegung in die Spannposition, also bis zur Blockierung durch das Sperrelement 8 aus.

Diese Vorbewegung, die vorzugsweise in die gleiche Richtung wie die bereits angesprochene Hauptbewegung H während des normalen Zerstäubungsvorgangs verläuft, führt zur Erzeugung und Ausgabe der gewünschten Vormenge an Fluid 2.

Die Vorbewegung ist im Vergleich zur Hauptbewegung sehr kurz. Insbesondere beträgt ihr Hub d - also die Strecke von der Überspannposition in die Spannposition - nur etwa 0,2 bis 1 mm, insbesondere etwa 0,4 bis 0,8 mm.

Bedarfsweise ist der Hub d zur Anpassung an die Düse 12, den Druckerzeuger 5 oder dergleichen und/oder zur Anpassung an das jeweilige Fluid 2 einstellbar bzw. festlegbar. Durch Variation des Hubs d ist entsprechend das Vormengenvolumen variierbar.

Je nach Vormengenvolumen und Druckanstieg - insbesondere Rücklaufgeschwindigkeit des Förderelements bzw. Förderrohrs 9 von der Überspannposition in die Spannposition - und je nach Fluid 2 erfolgt eine Ausgabe der Vormenge als Tropfen und/oder als Sprühnebel bzw. Sprühstrahl. Der Benutzer kann den Tropfen und eventuell auch sonstige bei der normalen Benutzung des Zerstäubers 1 an der Düse 12 verbleibende Restmengen von Fluid 2 bedarfsweise abwischen oder abschütteln. Alternativ oder zusätzlich kann auch ein Abstreifen oder Aufnehmen des Tropfens bzw. der Restmengen durch eine nicht dargestellte Abdeckkappe zum Verschließen des Mundstücks 13, insbesondere durch ein Abstreif- oder Aufnahmemittel, wie ein Vlies oder dergleichen, in der Abdeckkappe aufgenommen werden.

Die Abdeckkappe kann zumindest bereichsweise transparent ausgebildet sein, so daß der Benutzer die Ausgabe der Vormenge verfolgen kann. Alternativ ist es auch möglich, daß der Benutzer die Ausgabe der Vormenge nicht verfolgt und insbesondere auch nicht bemerkt. Die Ausgabe der Vormenge kann nämlich quasi unmerklich und derart schnell erfolgen, daß dies die normale Handhabung und Benutzung des Zerstäubers 1 für die Ausgabe und Zerstäubung von Fluid 2 nicht beeinträchtigt.

Gemäß einer nicht dargestellten Weiterbildung kann eine zusätzliche Sperre oder Verzögerung vorgesehen sein, so daß eine Betätigung oder Auslösung des gespannten Zerstäubers 1 - also des Sperrelements 8 bzw. der Taste 23 beim Darstellungsbeispiel - erst dann möglich ist, wenn die Vormenge an Fluid 2 ausgegeben worden ist bzw. die Halterung 6 und das Förderrohr 9 die Spannposition erreicht haben, also die Halterung 6 am Sperrelement 8 anliegt. So kann verhindert werden, daß die Vormenge zusammen mit der eigentlichen Dosis an Fluid 2 ausgegeben und zerstäubt wird, also die normale Dosis in unerwünschter Weise um die Vormenge erhöht wird.

Nachdem die Vormenge ausgegeben wurde, kann der Benutzer den Zerstäuber 1 ganz normal betätigen, insbesondere durch Drücken der Taste 23, um das Sperrelement 8 aus der Sperrlage in die die Halterung 6 nicht blockierende, beim Darstellungsbeispiel konzentrische Lage zu verlagern. Damit wird die Halterung 6 freigegeben und es erfolgt die normale Ausgabe und Zerstäubung. Aufgrund der Federkraft der Antriebsfeder 7 führt das Förderrohr 9 die Hauptbewegung H aus, wobei in der Druckkammer 11 befindliches Fluid 2 über die Düse 12 ausgegeben und zerstäubt wird.

Überraschenderweise wurde festgestellt, daß sich die gezielt eingestellte Vormenge zur Spülung der Düse 12 bzw. des gesamten Düsen- oder Ausgabesystems nutzen läßt, um insbesondere Verlegungen, Ablagerungen, Verschmutzungen oder mikrobielle Verunreinigungen unmittelbar vor einer Verwendung des Zerstäubers 1, also unmittelbar insbesondere vor einer Inhalation, austragen bzw. auflösen zu können. Das vorschlagsgemäße Spülen mit der Vormenge verringert auch das Risiko einer Düsenverstopfung durch Ablagerungen, Verschmutzungen, Auskristallisationen oder dergleichen.

Weiter können unerwünschte Konzentrationszunahmen, insbesondere im Düsenbereich, vermieden oder zumindest minimiert werden. Derartige Aufkonzentrationen - beispielsweise eine unerwünschte Zunahme eines Wirkstoffs oder sonstigen Stoffs - treten nämlich insbesondere bei lösemittelhaltigen Fluiden 2, besonders stark bei ethanolischen oder ethanolhaltigen Fluiden 2, auf. Die Verhinderung oder Verminderung von Aufkonzentrationen im Düsenbereich trägt auch zu einer höheren Dosiergenauigkeit bei.

Die Dosiergenauigkeit kann des weiteren auch dadurch verbessert werden, daß die Vormenge Teile des Druckerzeugers 5 bzw. Düsensystems bereits vor der eigentlichen Benutzung benetzt.

Ein besonderer Vorteil des vorschlagsgemäßen Spülens mit der Vormenge liegt darin, daß dies bei jeder Betätigung, insbesondere jedem Spannen, des Zerstäubers 1 - insbesondere also zwangsweise - erfolgt und nicht vom Benutzer beeinflußt werden kann oder muß.

Das vorschlagsgemäße Spülen kann auch dazu verwendet werden, den mikrobiologischen Zustand des Inhalators weiter zu verbessern. Insbesondere werden eventuell entstehende Keime ausgetragen. Falls oligodynamische Silberverbindungen im Zerstäuber 1 eingesetzt werden, können durch das Spülen mit der Vormenge entstehende Silberionen ausgebracht werden, so daß weniger Silberionen ausgegeben und inhaliert werden.

Das vorschlagsgemäße Spülen mit der Vormenge bewirkt ferner, daß das Ventil 10 in eine definierte Schließstellung gebracht wird, so daß bei der nachfolgenden eigentlichen Betätigung des Zerstäubers 1 das Fluid 2 sofort unter Druck gesetzt und austragen wird. Die Schließzeit des Ventils 10 wird nämlich verringert und stabilisiert. Hierdurch wird die Variabilität des abgegebenen Volumens verringert, kann also eine weitere Verbesserung der Dosiergenauigkeit erreicht werden.

Die vorgenannten Effekte und Vorteile lassen sich mit einer überraschend geringen bzw. kleinen Vormenge erreichen, insbesondere mit den oben genannten Volumina bzw. Verhältnissen.

Es ist anzumerken, daß das vorschlagsgemäße Spülen vor der eigentlichen Fluidausgabe generell auch bei sonstigen Zerstäubern eingesetzt werden kann. Insbesondere sind auch verschiedene konstruktive Lösungen und Varianten zum vorschlagsgemäßen Spülen bzw. zur Erzeugung der Vormenge einsetzbar. Weiter sind auch verschiedene andere konstruktive Lösungen zur Sperrung und Entsperrung in der Spannposition möglich. Generell kann anstelle der Antriebsfeder 7 auch ein sonstiges Feder- oder Antriebsmittel eingesetzt werden. Selbst bei manuellem Antrieb des Förderelements in Haupt- bzw. Ausgaberichtung kann die Erzeugung und Ausgabe der Vormenge zum Spülen realisiert werden.

Im Gegensatz zu Standgeräten oder dergleichen ist der vorschlagsgemäße Zerstäuber 1 vorzugsweise transportabel ausgebildet, insbesondere handelt es sich um ein mobiles Handgerät.

Die vorschlagsgemäße Lösung kann aber nicht nur bei den hier im einzelnen beschriebenen Zerstäubern 1 sondern auch bei sonstigen Zerstäubern oder Inhalatoren, beispielsweise Pulverinhalatoren oder sogenannten "metered dose inhalers", eingesetzt werden.

Besonders bevorzugt ist der Zerstäuber 1 als Inhalator, insbesondere zur medizinischen Aerosol-Therapie, ausgebildet. Alternativ kann der Zerstäuber 1 jedoch auch für sonstige Zwecke ausgebildet sein, vorzugsweise der Zerstäubung einer kosmetischen Flüssigkeit dienen und insbesondere als Parfümzerstäuber ausgebildet sein. Der Behälter 3 enthält entsprechend beispielsweise eine Arzneimittelformulierung oder eine kosmetische Flüssigkeit, wie Parfüm oder dgl.

Vorzugsweise handelt es sich bei dem Fluid 2 um eine Flüssigkeit, wie bereits erwähnt, insbesondere um eine wäßrige oder ethanolische Arzneimittelformulierung. Es kann sich jedoch auch um eine sonstige Arzneimittelformulierung, eine Suspension oder dgl. oder auch um Partikel oder Pulver handeln.

Nachfolgend werden bevorzugte Bestandteile und/oder Formulierungen des vorzugsweise medizinischen Fluids 2 aufgeführt. Wie bereits erwähnt, kann es sich um wäßrige oder nicht wäßrige Lösungen, Mischungen, ethanolhaltige oder lösungsmittelfreie Formulierungen handeln. Besonders bevorzugt sind im Fluid 2 enthalten:

Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar, kombiniert mit einem Betamimetika, Corticosteroiden, PDE4-Inhibitoren, EGFR-Hemmern oder LTD4-Antagonisten,
- **W** stellt ein Corticosteroiden dar, kombiniert mit einem PDE4-Inhibitoren, EGFR-Hemmern oder LTD4-Antagonisten
- **W** stellt ein PDE4-Inhibitoren dar, kombiniert mit einem EGFR-Hemmern oder LTD4-Antagonisten
- **W** stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HO-KU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.
Ebenfalls bevorzugte Anticholinergika sind ausgewählt aus den Salzen der Formel **AC-1** 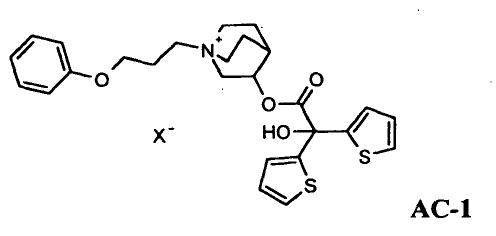 worin X ⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 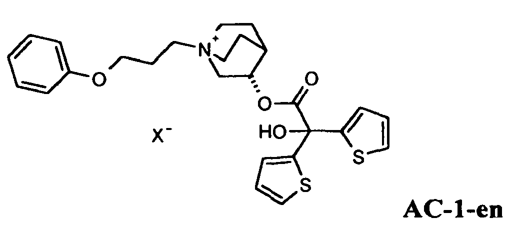 enthalten, worin X ⁻ die vorstehend genannten Bedeutungen aufweisen kann. Weiterhin bevorzugte Anticholinergika sind ausgewählt aus den Salzen der Formel **AC-2** 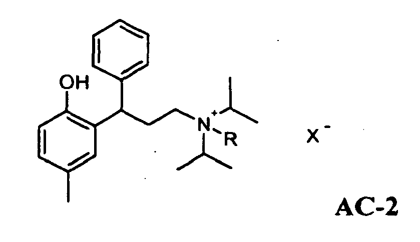 worin R entweder Methyl oder Ethyl bedeuten und worin X ⁻ die vorstehend genannte Bedeutungen aufweisen kann. In einer alternativen Ausfiihrungsform kann die Verbindung der Formel **AC-2** auch in Form der freien Base **AC-2-base** vorliegen.

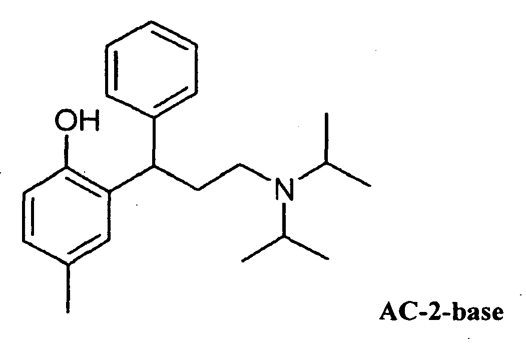

Weiterhin genannte Verbiridungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methylisothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(tert-butyl)-9H-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2- hydroxy-2-propyl)phenyl)thio)methylcyclopropan-essigsäure,
- 1-(((1(R)-3(3-(2-(2,3-Dichlorthieno[3,2-b]pyridin-5-yI)-(E)-ethenyl)phenyl)-3-(2-(1-hydroxy-1-methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)aminol-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazol in
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 A1 oder CA 2297174 A1 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

### Bezugszeichenliste

- 1: Zerstäuber
- 2: Fluid
- 3: Behälter
- 4: Fluidraum
- 5: Druckerzeuger
- 6: Halterung
- 7: Antriebsfeder
- 8: Sperrelement
- 9: Förderrohr
- 10: Rückschlagventil
- 11: Druckkammer
- 12: Düse
- 13: Mundstück
- 14: Aerosol
- 15: Zuluftöffnung
- 16: Gehäuseoberteil
- 17: Innenteil
- 17a: oberes Teil des Innenteils
- 17b: unteres Teil des Innenteils
- 18: Gehäuseteil (Unterteil)
- 19: Halteelement
- 20: Getriebe
- 21: Gleitfläche
- 22: Gleitfläche
- 23: Taste

- H: Hauptbewegung
- S: Spannbewegung
- d: Hub

## Patentansprüche

1. Zerstäuber (1) für ein Fluid (2), mit einem Fluidreservoir, insbesondere einem Behälter (3), ein zur Fluidförderung bewegbares Förderelement; und mit einer Düse (12) zur Ausgabe und Zerstäubung von Fluid (2), wobei das Fluid (2) dosiert in einzelnen Dosen ausgebbar ist,
**dadurch gekennzeichnet,**
**daß** der Zerstäuber (1) derart ausgebildet, daß eine die Düse (12) spülende Vormenge an Fluid (2) vor jeder bestimmungsgemäßen Ausgabe einer Dosis oder bei jedem Spannen des Zerstäubers (1) ausgegeben wird, wobei das Fluid mittels eines bewegbaren Förderelementes gefördert wird, indem das Förderelement von einer Überspann- in eine Spannposition bewegbar ist.

2. Zerstäuber nach Anspruch 1, **dadurch gekennzeichnet, daß** die Vormenge mindestens 0,5 µl, insbesondere 0,5 µl bis 3 µl, beträgt.

3. Zerstäuber nach Anspruch 1 oder 2, **dadurch gekennzeichnet, daß** die Vormenge mindestens 1%, vorzugsweise mindestens 2%, insbesondere etwa 3 bis 10%, der Menge einer Dosis beträgt und/oder mindestens das 5-fache des Volumens der Düse (12) beträgt.

4. Zerstäuber nach einem der voranstehenden Ansprüche, **dadurch gekennzeichnet, daß** der Zerstäuber (1) ein zur Fluidförderung bewegbares Förderelement, insbesondere ein Förderrohr (9), aufweist, das zunächst in einer Vorbewegung zur Ausgabe der Vormenge - insbesondere bis zu einer Spannposition - und dann in einer Hauptbewegung zur Ausgabe der Dosis bzw. eigentlichen Ausgabe und Zerstäubung von Fluid (2) bewegbar ist.

5. Zerstäuber nach Anspruch 4, **dadurch gekennzeichnet, daß** die Vorbewegung und/oder Hauptbewegung vorzugsweise ausschließlich durch Federkraft erfolgt bzw. erfolgen.

6. Zerstäuber nach Anspruch 4 oder 5, **dadurch gekennzeichnet, daß** das Förderelement gegen Federkraft - insbesondere mittels eines Getriebes (20) und/oder durch manuelle Betätigung des Zerstäubers (1) - in eine Überspannposition bewegbar ist, aus der das Förderelement durch die Federkraft in die Spannposition bewegbar ist.

7. Zerstäuber nach einem der Ansprüche 4 bis 6, **dadurch gekennzeichnet, daß** der Hub (d) der Vorbewegung 0,2 bis 1 mm, insbesondere 0,4 bis 0,8 mm, beträgt.

8. Zerstäuber nach einem der Ansprüche 4 bis 7, **dadurch gekennzeichnet, daß** der Zerstäuber (1) ein Sperrelement (8) aufweist, das in einer Sperrlage das Förderelement mittelbar oder unmittelbar in der Spannposition sperrt und insbesondere manuell aus der Sperrlage zur Freigabe des Förderelements verlagerbar ist.

9. Zerstäuber nach einem der voranstehenden Ansprüche, **dadurch gekennzeichnet, daß** der Zerstäuber (1) ein lösemittelhaltiges, insbesondere ethanolhaltiges Fluid (2) enthält.

10. Zerstäuber nach einem der voranstehenden Ansprüche, **dadurch gekennzeichnet, daß** der Zerstäuber (1) eine Inhalationsformulierung oder ein Medikament als Fluid (2) enthält, insbesondere ausgewählt aus der Gruppe bestehend aus Anticholinergika, Betamimetika, Steroiden, Phosphodiesterase IV-inhibitoren, LTD4-Antagonisten und EGFR-Kinase-Hemmer, Antiallergika, Derivate von Mutterkornalkaloiden, Triptane, CGRP-Antagonisten, Phosphodiesterase-V-Inhibitoren, sowie Kombinationen aus solchen Wirkstoffen, z.B. Betamimetika plus Anticholinergika oder Betamimetica plus Antiallergika.

11. Zerstäuber nach einem der voranstehenden Ansprüche, **dadurch gekennzeichnet, daß** der Zerstäuber (1) als vorzugsweise tragbarer Inhalator, insbesondere zur medizinischen Aerosol-Therapie, ausgebildet ist.

12. Verfahren zur Ausgabe und Zerstäubung von Fluid (2) mit einem zur Fluidförderung bewegbarem Förderelement, wobei das Fluid, dosiert in einzelnen Dosen durch eine Düse (12) ausgeben wird,
**dadurch gekennzeichnet,**
**daß** eine die Düse (12) spülende Vormenge an Fluid (2) vor jeder bestimmungsgemäßen Ausgabe einer Dosis oder bei jedem Spannen des Zerstäubers (1) ausgegeben wird, wobei das Fluid mittels eines bewegbaren Förderelementes gefördert wird, indem das Förderelement von einer Überspann- in eine Spannposition bewegbar ist.

13. Verfahren nach Anspruch 12, **dadurch gekennzeichnet, daß** die Vormenge mindestens 0,5 µl, insbesondere 0,5 µl bis 3 µl, beträgt.

14. Verfahren nach Anspruch 12 oder 13, **dadurch gekennzeichnet, daß** die Vormenge mindestens 1%, vorzugsweise mindestens 2%, insbesondere etwa 3 bis 10%, der Menge einer Dosis beträgt.

15. Verfahren nach einem der Ansprüche 12 bis 14, **dadurch gekennzeichnet, daß** ein zur Fluidförderung bewegbares Förderelement, insbesondere ein Förderrohr (9), zunächst in einer Vorbewegung zur Ausgabe der Vormenge - insbesondere bis zu einer Spannposition - und dann in einer Hauptbewegung zur Ausgabe der Dosis bzw. eigentlichen Ausgabe und Zerstäubung von Fluid (2) bewegt wird.

## Claims

1. Atomiser (1) for a fluid (2), having a fluid reservoir, in particular a container (3), a conveying element which is movable for conveying fluid, and having a nozzle (12) for the delivery and atomisation of fluid (2), wherein the fluid (2) can be delivered in metered individual doses,
**characterised in that**
the atomizer (1) is constructed so that a preliminary amount of fluid (2) that rinses the nozzle (12) is delivered before each intended delivery of a dose or on each tensioning of the atomizer (1), the fluid being conveyed by means of a movable conveying element, by the conveying element moving from an over-tensioning position into a tensioning position.

2. Atomiser according to Claim 1, **characterised in that** the preliminary amount is at least 0.5 µl, in particular 0.5 µl to 3 µl.

3. Atomiser according to Claim 1 or 2, **characterised in that** the preliminary amount is at least 1%, preferably at least 2%, in particular about 3 to 10%, of the amount of a dose and/or at least five times the volume of the nozzle (12).

4. Atomiser according to one of the preceding claims, **characterised in that** the atomizer (1) has a conveying element, particularly a conveying tube (9), which is movable for conveying fluid, which is movable first of all in a preliminary movement for delivering the preliminary amount - in particular to a tensioning position - and then in a main movement for delivering the dose or for the actual delivery and atomisation of fluid (2).

5. Atomiser according to Claim 4, **characterised in that** the preliminary movement and/or main movement is or are preferably carried out exclusively by spring force.

6. Atomiser according to Claim 4 or 5, **characterised in that** the conveying element is movable counter to spring force - in particular by means of a gear (20) and/or by manual actuation of the atomizer (1) - into an over-tensioning position from which the conveying element can be moved by spring force into the tensioning position.

7. Atomiser according to one of Claims 4 to 6, **characterised in that** the stroke (d) of the preliminary movement is 0.2 to 1 mm, in particular 0.4 to 0.8 mm.

8. Atomiser according to one of Claims 4 to 7, **characterised in that** the atomizer (1) has a locking element (8) which in a locking position secures the conveying element directly or indirectly in the tensioning position and in particular can be moved manually out of the locking position in order to release the conveying element.

9. Atomiser according to one of the preceding claims, **characterised in that** the atomizer (1) contains a fluid (2) that contains a solvent, in particular contains ethanol.

10. Atomiser according to one of the preceding claims, **characterised in that** the atomizer (1) contains an inhalable formulation or a medicament in the form of a fluid (2), selected in particular from among the anticholinergics, betamimetics, steroids, phosphodiesterase IV-inhibitors, LTD4-antagonists and EGFR-kinase-inhibitors, antiallergics, ergot alkaloid derivatives, triptans, CGRP-antagonists, phosphodiesterase-V-inhibitors, and combinations of such active substances, e.g. betamimetics plus anticholinergics or betamimetics plus antiallergics.

11. Atomiser according to one of the preceding claims, **characterised in that** the atomizer (1) is constructed as a preferably portable inhaler, in particular for medicinal aerosol therapy.

12. Process for delivering and atomising fluid (2), having a conveying element which is movable for conveying fluid, wherein the fluid (2) is delivered in individual metered doses through a nozzle (12),
**characterised in that**
a preliminary amount of fluid (2) that rinses the nozzle (12) is delivered before each intended delivery of a dose or on each tensioning of the atomizer (1), the fluid being conveyed by means of a movable conveying element, by the conveying element moving from an over-tensioning position into a tensioning position.

13. Process according to Claim 12, **characterised in that** the preliminary amount is at least 0.5 µl, in particular 0.5 µl to 3 µl.

14. Process according to Claim 12 or 13, **characterised in that** the preliminary amount is at least 1%, preferably at least 2%, in particular about 3 to 10%, of the amount of a dose.

15. Process according to one of Claims 12 to 14, **characterised in that** a conveying element, particularly a conveying tube (9), which is movable for conveying fluid, is moved first of all in a preliminary movement for delivering the preliminary amount - in particular to a tensioning position - and then in a main movement for delivering the dose or for the actual delivery and atomisation of fluid (2).

## Revendications

1. Pulvérisateur (1) pour un fluide (2), avec un réservoir à fluide, en particulier un réservoir (3), un élément de transport mobile pour le transport du fluide, et avec une buse (12) pour l'émission et la pulvérisation du fluide (2), le fluide (2) pouvant être débité en doses individuelles,
**caractérisé en ce que**
le pulvérisateur (1) est configuré de telle sorte qu'une quantité préliminaire de fluide (2) rinçant la buse (12) est émise avant chaque émission d'une dose conforme au but ou avant chaque mise en charge du pulvérisateur (1), le fluide étant transporté au moyen d'un élément de transport mobile **en ce que** l'élément de transport peut être déplacé d'une position de surcharge à une position de charge.

2. Pulvérisateur selon la revendication 1, **caractérisé en ce que** la quantité préliminaire est d'au moins 0,5 µl, en particulier entre 0,5 µl et 3 µl.

3. Pulvérisateur selon la revendication 1 ou 2, **caractérisé en ce que** la quantité préliminaire est d'au moins 1 %, de préférence d'au moins 2 %, en particulier d'environ 3 à 10 % de la quantité d'une dose et/ou d'au moins 5 fois le volume de la buse (12).

4. Pulvérisateur selon l'une quelconque des revendications précédentes, **caractérisé en ce que** le pulvérisateur (1) comporte un élément de transport mobile pour le transport du fluide, en particulier un tube de transport (9), qui peut d'abord être déplacé dans un mouvement préliminaire pour l'émission de la quantité préliminaire - en particulier jusqu'à une position de charge - et ensuite dans un mouvement principal pour l'émission de la dose, respectivement pour l'émission et la pulvérisation proprement dites du fluide (2).

5. Pulvérisateur selon la revendication 4, **caractérisé en ce que** le mouvement préliminaire et/ou le mouvement principal s'effectue(nt) de préférence exclusivement par la force d'un ressort.

6. Pulvérisateur selon la revendication 4 ou 5, **caractérisé en ce que** l'élément de transport peut être déplacé dans une position de surcharge, contre la force d'un ressort - en particulier au moyen d'un engrenage (20) et/ou par l'actionnement manuel du pulvérisateur (1) - position de laquelle l'élément de transport peut être déplacé par la force du ressort dans la position de charge.

7. Pulvérisateur selon l'une quelconque des revendications 4 à 6, **caractérisé en ce que** la course (d) du mouvement préliminaire est de 0,2 à 1 mm, en particulier de 0,4 à 0,8 mm.

8. Pulvérisateur selon l'une quelconque des revendications 4 à 7, **caractérisé en ce que** le pulvérisateur (1) comporte un élément de blocage (8) qui, dans une position de blocage, bloque indirectement ou directement l'élément de transport dans la position de charge et qui est en particulier déplaçable manuellement depuis la position de blocage pour la libération de l'élément de transport.

9. Pulvérisateur selon l'une quelconque des revendications précédentes, **caractérisé en ce que** le pulvérisateur (1) contient un fluide (2) contenant un solvant, en particulier contenant de l'éthanol.

10. Pulvérisateur selon l'une quelconque des revendications précédentes, **caractérisé en ce que** le pulvérisateur (1) contient une formulation à inhaler ou un médicament comme fluide (2), choisi en particulier parmi le groupe consistant en des anticholinergiques, des bêtamimétiques, des stéroïdes, des inhibiteurs de phosphodiestérase IV, des antagonistes de LTD 4 et des inhibiteurs de kinase de l'EGFR, des antiallergiques, des dérivés des alcaloïdes de l'ergot de seigle, des triptanes, des antagonistes de CGRP, des inhibiteurs de phosphodiestérase V, ainsi que des combinaisons de tels principes actifs, par exemple bêtamimétiques plus antichoninergiques ou bêtamimétiques plus antiallergiques,

11. Pulvérisateur selon l'une quelconque des revendications précédentes, **caractérisé en ce que** le pulvérisateur (1) est configuré comme inhalateur de préférence portable, en particulier pour la thérapie médicale par aérosols.

12. Procédé d'émission et de pulvérisation de fluide (2) avec un élément de transport mobile pour le transport du fluide, le fluide étant émis par une buse (12), dosé en doses individuelles,
**caractérisé en ce que**
une quantité préliminaire de fluide (2) rinçant la buse (12) est émise avant chaque émission d'une dose conforme au but ou avant chaque mise en charge du pulvérisateur (1), le fluide étant transporté au moyen d'un élément de transport mobile **en ce que** l'élément de transport peut être déplacé d'une position de surcharge à une position de charge.

13. Procédé selon la revendication 12, **caractérisé en ce que** la quantité préliminaire est d'au moins 0,5 µl, en particulier entre 0,5 µl et 3 µl.

14. Procédé selon la revendication 12 ou 13, **caractérisé en ce que** la quantité préliminaire est d'au moins 1 %, de préférence d'au moins 2 %, en particulier d'environ 3 à 10 % de la quantité d'une dose.

15. Procédé selon l'une quelconque des revendications 12 à 14, **caractérisé en ce qu'**un élément de transport mobile pour le transport du fluide, en particulier un tube de transport (9), est d'abord déplacé dans un mouvement préliminaire pour l'émission de la quantité préliminaire - en particulier jusqu'à une position de charge - et ensuite dans un mouvement principal pour l'émission de la dose, respectivement pour l'émission et la pulvérisation proprement dites du fluide (2).
